Europäisches Patentamt

European Patent Office

Office européen des brevets

(11) Publication number: **0 321 601**

**A1**

## EUROPEAN PATENT APPLICATION

(21) Application number: 87119080.7

(22) Date of filing: 22.12.87

(51) Int. Cl.⁴ **C12N 15/00 , C12N 5/00 , A61K 37/02 , C12P 21/02**

Claims 11 - 24 are deemed to be abandoned due to non-payment of the claims fees (Rule 31 (2) EPC).

(43) Date of publication of application:
**28.06.89 Bulletin 89/26**

(84) Designated Contracting States:
**GB**

(71) Applicant: **Kishimoto, Tadamitsu, Prof.**
**Division of Cellular Immunology Institute for**
**Molecular and Cellular Biology University**
**Osaka**
**1-3, Yamadaoka Suita Osaka 565(JP)**

(72) Inventor: **Kishimoto,Tadamitsu,Prof. Dr.**
**3-5-31, Nakano, Tondabayashi**
**Osaka 584(JP)**
Inventor: **Suemura,Masaki,Dr.**
**9-5, Himemuro, Ikeda**
**Osaka 563(JP)**
Inventor: **Kikutani,Hitoshi,Dr.**
**2-17-B504, Senriyama-Higashi, Suita**
**Osaka 565(JP)**
Inventor: **Barsumian,Edward L.,Dr.**
**3-11-5-503, Senba-Nishi, Mino**
**Osaka 562(JP)**

(74) Representative: **Laudien, Dieter, Dr. et al**
**Boehringer Ingelheim Zentrale GmbH ZA**
**Patente Postfach 200**
**D-6507 Ingelheim am Rhein(DE)**

(54) Soluble recombinant Fc-Epsilon-receptor, the preparation and the use thereof.

(57) The present invention describes the expression of highly bioactive water-soluble $Fc_\epsilon R$ in cells derived from multi-cellular organismns and the use thereof.

EP 0 321 601 A1

## Soluble recombinant Fcε-receptor, the preparation and the use thereof

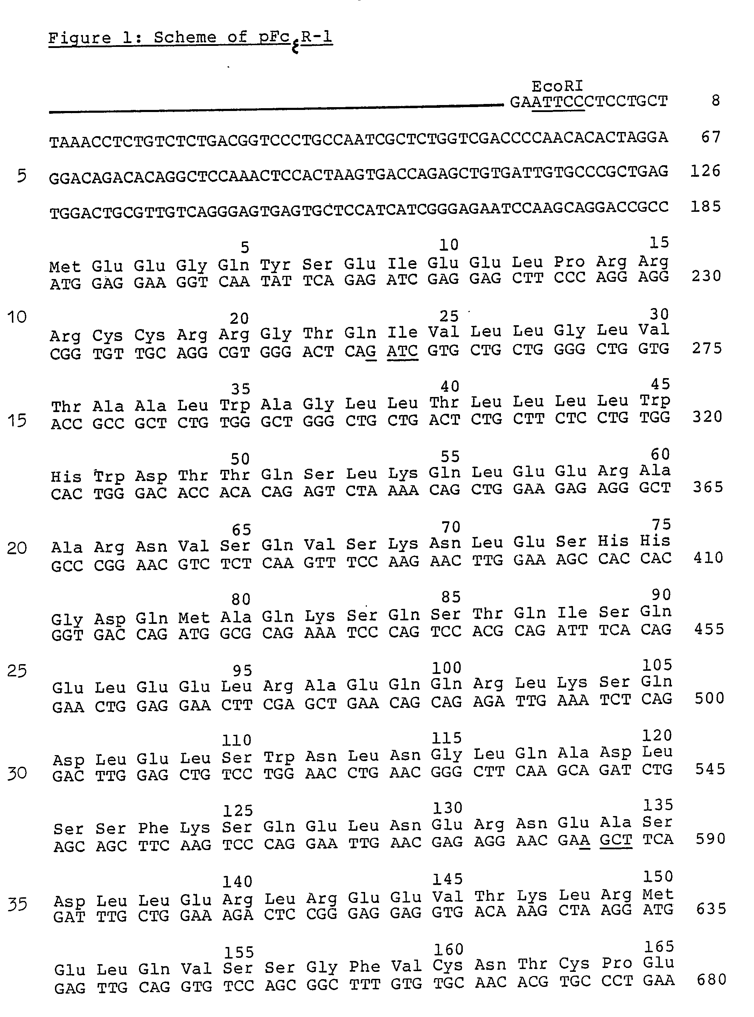

The molecular structure of Human Lymphocyte Receptor for Immunoglobulin (FcεR) as well as its cloning and expression was recently described in the literature by Tadamitsu Kishimoto et al. (see Cell 47, 657-665 (1986)) and by Guy Delespesse et al. (see The Embo Journal 6, 109-114 (1987)). The molecular structure of FcεR (see Figure 1) indicates that it is oriented on the cell membrane with its N-terminus inside the cell and its carboxy terminus exposed outside the cell.

Furthermore, the residue at position 150 of FcεR is a potential site for trypsin-like proteases, which explains the presence of soluble components of FcεR in culture supernatants of B cells and certain B lymphoblastoid cell lines. This also explains the presence of water-soluble FcεR or a portion of it associated or complexed to IgE in the serum of normal and atopic individuals, with significantly higher levels in the latter group.

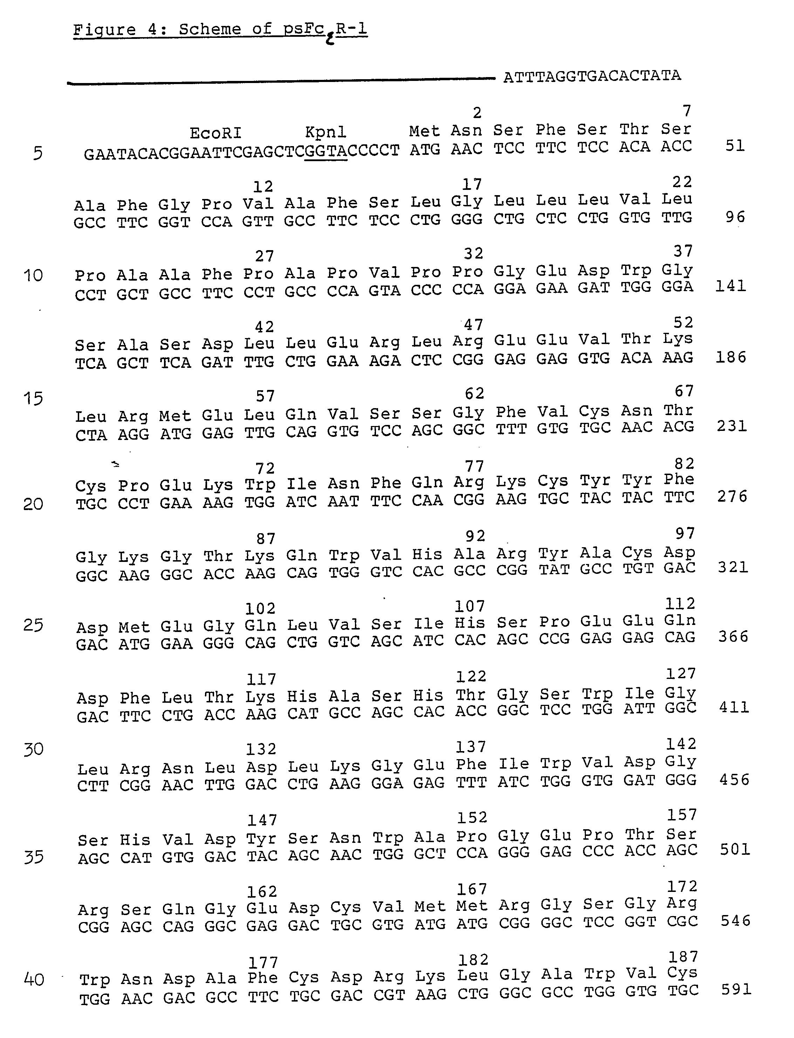

Moreover, in the not yet published European Patent Application Nr. 87111392.4 are described a cDNA-sequence, wherein at least a part of the cDNA sequence coding for the amino acids 1 to 148 is replaced by a suitable cDNA fragment coding for an eucaryotic signal sequence. Thus, in the described plasmid psFcεR-1 (see Figure 4) the coding sequence for the amino acids 1 to 133 is replaced by the BSF-2 signal sequence as follows:

The plasmid LE392 (see Cell 47, 657-665 (1986)) deposited under the terms of the Budapest Treaty under FERM BP-1116 on August 01, 1986 was digested with HindIII, whereby a 1.0 kbp HindIII-fragment was obtained containing the coding sequence for the amino acids 134 to 321 of the full-length FcεR cDNA. The recessed 3′-ends of this fragment were then filled in with the Klenow fragment of DNA polymerase and the DNA subsequently digested with PstI. The obtained fragment was then cloned in a suitable vector, preferably with a BamHI-PstI digested pBSF$_2$-L8, whereby this vector is conveniently prepared as follows:

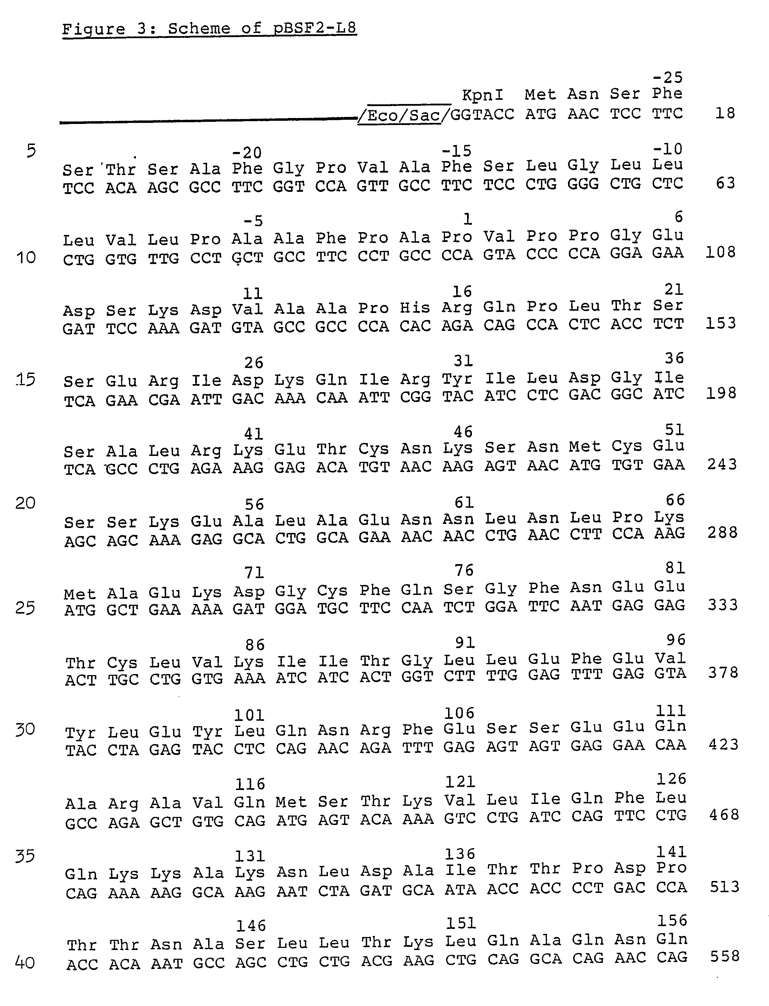

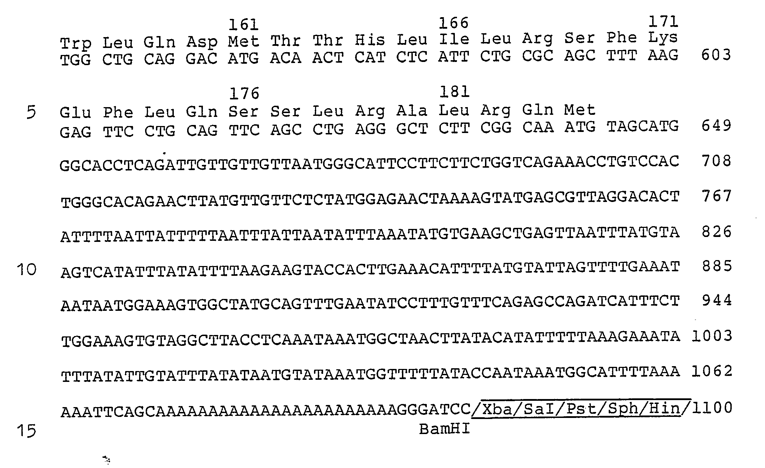

The EcoRI-BamHI 1,2 kbp BSF-2 cDNA insert was prepared by digestion of pBSF-2.38 (see Nature 324, 73-76 (1986)) with HindIII and BamHI. The obtained fragment containing a full length BSF-2 cDNA was then digested with HinfI and the recessed 3′-end filled in with Klenow fragment of DNA polymerase. After KpnI digestion, the obtained KpnI-HinfI 110 bp fragment containing the BSF-2 leader sequence was cloned into the multiple cloning site of PGEM4 digested previously with KpnI and SmaI. One of the selected clones was propagated and named as pBSF2-L8 (see Figure 3).

pBSF2-L8 was digested with BamHI and the recessed 3′-ends filled in with Klenow fragment of DNA polymerase. After the filling in of the BamHI site, the above mentioned HindIII-PstI FcεR cDNA was cloned into BamHI-PstI digested pBSF2-L8 as mentioned hereinbefore. One of the selected clones was propagated and named as psFcεR-1 (see Figure 4).

Surprisingly it was found, that when expressing such a coding sequence wherein at least a part of the cDNA sequence coding for the amino acids 1 to 148 is replaced by a suitable cDNA fragment coding for an eucaryotic signal sequence, in cells derived from multicellular organisms a highly bioactive water-soluble FcεR is obtained. Hereby, in principle, any cell culture is workable, whether using vertebrate or invertebrate cells. However, interest has been greatest in vertebrate cells, and propagation of vertebrate cells in culture (tissue culture) has become a routine procedure in recent years (Tissue Culture, Academic Press, Kruse and Patterson, Editors (1973)). Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promotor located in front of the gene to be expressed, along with any necessary ribosome binding sites, RNA splice sites, polyadenylation site, and transcriptional terminator sequences.

For use in mammalian cells, the control functions on the expression vectors are often provided by viral genome. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273, 113 (1978)). Smaller or larger SV40 fragments may also be used, provided there is included the approximately 250 bp sequence extending from the Hind III site toward the Bgl I site location in the viral origin of replication. Further, it is also possible, and often desirable, to utilize promotor or control sequences normally associated with the desired gene sequence, provided such control sequences are compatible with the host cell systems.

An origin of replication may be provided either by construction of the vector to include an exogenous origin, such as may be derived from SV40 or other viral (e.g., Polyoma, Adeno, VSV, BPV, etc.) source, or may be provided by the host cell chromosomal replication mechanism. If the vector is integrated into the host cell chromosome, the latter is often sufficient.

However, most preferably a cloning vehicle (shuttle plasmid) is used which enables replication in eukaryotes as well as in prokaryotes. The plasmid's ability to replicate in prokaryotes provides easy means for manipulating the DNA sequence and getting hold of large quantities of plasmid DNA needed for transfection into mammalian cells.

Such a shuttle plasmid contains prokaryotic DNA motifs as well as DNA sequences derived from eukaryotes.

The prokaryotic part of the plasmid consists of an origin of replication usually derived from the plasmid pBR322 (Mulligan R.C. et al. in Proc. Natl. Acad. Sci. USA 78, 2072-2076 (1981)) and a marker gene facilitating selection on antibiotic containing medium. The most widely used genes for selection are those mediating resistance to either ampicillin, tetracycline or chloramphenicol (Mulligan R.C. et al. in Proc. Natl. Acad. Sci. USA 78, 2072-2076 (1981)).

The eukaryotic part of the shuttle plasmid has to contain an origin of replication, usually derived from viral genomes such as Simian 40 Virus (Mulligan R.C. et al. in Proc. Natl. Acad. Sci. USA 78, 2072-2076 (1981)) or Bovine Papilloma Virus (DiMaio D. et al. in Mol. Cell. Biol. 4, 340-350 (1984)). Secondly a selectable marker gene is required to enable the cells harbouring the shuttle plasmid to grow under selective conditions in order to maintain the plasmid in the cells. This marker gene may be either of prokaryotic or of eukaryotic origin (e.g. prokaryotic genes: gpt gene coding for xanthine-guanine phosphoribosyltransferase (Mulligan R.C. et al. in Proc. Natl. Acad. Sci. USA 78, 2072-2076 (1981)), Mulligan R.C. et al. in Science 209, 1422 (1980)), neo gene coding for a bacterial phosphatase mediating resistance to the neomycin derivative G418 (Southern P. et al. in J. Mol. Appl. Genet. 1, 327 (1982), Scholer U. et al. in Cell 36, 1422 (1984), CAT gene coding for the chloramphenicol acetyl- transferase (Gorman C. in Mol. Cell. Biol. 2, 1044 (1982)); eukaryotic genes: gene coding for the thymidine kinase (Wigler M. et al. in Cell 11 223 (1977)). The third eukaryotic DNA motif enabling expression of the cloned gene of interest is a promoter sequence, which may be either constitutive or inducible (e.g. constitutive promoter: simian 40 virus or rous sarcoma virus (Mulligan R.C. et al. in Science 209, 1422 (1980), Laimons L. et al. in Proc. Natl. Acad. Sci. USA 79, 6453 (1982)); inducible promoter: mouse mammary tumor virus promoter (Chapman A.B. et al. in Mol. Cell. Biol. 3, 1421-1429, heat shock protein promoter (Pelham H. et al. in EMBO J. 1, 1473 (1982)-), metallothionein promoter (Mayo K. et al. in Cell 29 99 (1982), Karin M. et al. in Nature 299, 797 (1982)).

One way to get hold of relatively high quantities of the soluble part of the $Fc_\epsilon$-receptor protein in higher eukaryotes is to anneal the soluble part of the $Fc_\epsilon$-receptor gene to the SV 40 promoter (constitutive) and clone this hybrid gene into a plasmid containing the gene coding for the dihydrofolate reductase (dhfr). Under selective pressure the dhfr gene and the adjacent DNA sequences are amplified up to a thousand times, elevating the yield not only of the dhfr gene but the soluble $Fc_\epsilon$-receptor part as well (EP-A-0 093 619).

A preferred embodiment of the present invention, however, is a vector suitable for expression in cells of multicellular organisms containing the above mentioned coding sequences of $psFc_\epsilon R$-1 as shown in Figure 4, the cells transfected with such a vector, the corresponding genes operably linked with a suitable replicon and control sequences, the expressed water-soluble $Fc_\epsilon R$ and processes of the preparation thereof.

For example, such a vector according to the invention can be prepared using as basic vector the PDE-2 as follows:

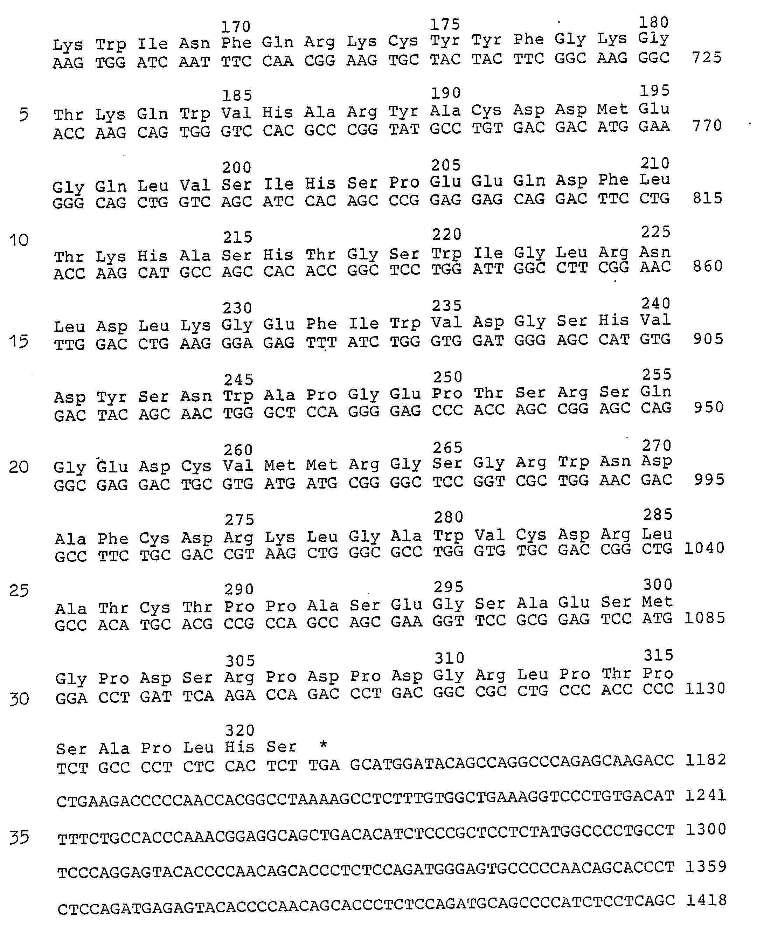

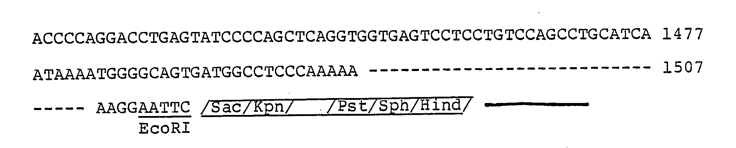

The expression vector PDE-2 (see Japanese Patent Publication 1986/88879) which bears two SV40 early promotors was digested with EcoRI using standard techniques, and ligated with the EcoRI fragment of $psFc_\epsilon R$-1 containing the BSF2 leader sequence (as shown on Figure 2). The resulting expression vector construct $PDE-2sFc_\epsilon R$ was utilised for expression in monkey Cos-7 cells.

In addition to microorganisms, cultures of cells derived from multicellular organisms may also be used as hosts. In principle, any such cell culture is workable, whether from vertebrate or invertebrate culture.

Propagation of cells in culture (tissue culture) has become a routine procedure (Tissue Culture, Academic Press, Kruse and Patterson, Editors (1973)). Examples of vertebrate host cell lines are VERO and Hela cells, Chinese hamster ovary (CHO) cell lines and WI38, BHK, Cos-7 and MDCK cell lines; but also invertebrate cell lines have become useful in recent years (e.g. 8f9 cloned cell line from Spodoptera frugiperda cells; available from ATCC).

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral genome. For example, commonly used promoters are derived from polyoma, Adenovirus 2, and most frequently Simian Virus 40 (SV40); for invertebrates e.g. the promoter from the polyhedrin gene of Autographa californica nuclear polyhedrosis virus (AcNPV) (Summers M.D. and Smith G.E.: A Manual of Methods for Baculovirus Vectors and Insect Cell Culture Procedures (1987) Department of Entomology, Texas, Agricultural Experiment Station and Texas A & M University).

The early and late promoters of SV40 are particularly useful because both are obtained easily from the virus as a fragment which also contains the SV40 viral origin of replication (Fiers et al., Nature 273 113 (1978)). Smaller or larger SV40 fragments may also be used, provided there is included the approximately 20 bp sequence extending from the HindIII site toward the BglI site location in the viral origin of replication. Further, it is also possible, and often desirable, to utilise promoter or control sequences normally associated with the desired gene sequence, provided such control sequences are compatible with the host cell systems (e.g. the DMFR-gene for CHO-cells).

An origin of replication may be provided either by construction of the vector to include an exogenous origin, such as may be derived from SV40 or other viral (e.g., Polyoma, Adeno, VSV, BPV, AcNPV etc.) source, or may be provided by the host cell chromosomal replication mechanisms. If the vector is integrated into the host cell chromosome, the latter is often sufficient.

However, most preferably a cloning vehicle (shuttle plasmid) is used which enables replication in eukaryotes as well as in prokaryotes. The plasmids's ability to replicate in prokaryotes provides easy means for manipulating the DNA sequence and getting hold of large quantities of plasmid DNA needed for transfection into mammalian cells.

Such a shuttle plasmid contains prokaryotic DNA motifs as well as DNA sequences derived from eukaryotes.

The prokaryotic part of the plasmid consists of an origin of replication usually derived from the plasmid pBR322 (Mulli gan R.C. et al. in Proc. Natl. Acad. Sci. USA 78, 2072-2076 (1981)) and a marker gene facilitating selection on antibiotic containing medium. The most widely used genes for selection are those mediating resistance to either ampicillin, tetracycline or chloramphenicol (Mulligan R.C. et al. in Proc. Natl. Acad. Sci. USA 78 2072-2076 (1981)).

The eukaryotic part of the shuttle plasmid has to contain an origin of replication, usually derived from viral genomes such as Simian 40 Virus (Mulligan R.C. et al. in Proc. Natl. Acad. Sci. USA 78, 2072-2076 (1981)) or Bovine Papilloma Virus (DiMaio D. et al. in Mol. Cell. Biol. 4, 340-350 (1984)). Secondly a selectable marker gene is required to enable the cells harbouring the shuttle plasmid to grow under selective conditions in order to maintain the plasmid in the cells. This marker gene may be either of prokaryotic or or eukaryotic origin (e.g. prokaryotic genes: gpt gene coding for xanthine-guanine phosphoribosyltransferase (Mulligan R.C. et al. in Proc. Natl. Acad. Sci USA 78, 2072-2076 (1981), Mulligan R.C. et al. in Science 209, 1422 (1980)), neo gene coding for a bacterial phosphatase mediating resistance to the neomycin derivative G418 (Southern P. et al. in J. Mol. Appl. Genet. 1, 327 (1982), Scholer U. et al. in Cell 36 1422 (1984)), CAT gene coding for the chloramphenicol acetyltransferase (Gormal C. in Mol. Cell. Biol. 2, 1044 (1982)), eukaryotic genes: gene coding for the thymidine kinase (Wigler M. et al. in Cell 11, 223 (1977)). The third eukaryotic DNA motif enabling expression of the cloned gene of interest is a promoter sequence, which may be either constitutive or inducible (e.g. constitutive promoter; simian 40 virus or rous sarcoma virus (Mulligan R.C. et al. in Science 209, 1422 (1980), Laimons L. et al. in Proc. Natl. Acad. Sci. USA 79, 6453 (1982)); inducible promoter: mouse mammary tumor virus promoter (Chapman A.B. et al. in Mol. Cell. Biol. 3, 1421-1429), heat shock protein promoter (Pelham H. et al. in EMBO J. 1 1473 (1982)), metallothionein promoter (Mayo K. et al. in Cell 29, 99 (1982), Karin M. et al. in Nature 299, 797 (1982)).

Transfer of foreign DNA into appropriate host cells can be managed by microinjection of DNA directly into the cell nucleus of the host cell (Capecchi M. in Cell 22, 479 (1980)); protoplast fusion of the bacterial protoplast carrying foreign DNA with the eucaryotic host cell (Schaffer W. in Proc. Natl. Acad. Sci. USA, 77 2163 (1980)); electroporation of the host cell membrane in the presence of DNA being transfected (Neuman E. et al. in EMBO J., 1, 841 (1982)); fusion of liposomes containing recombinant DNA with host cells (Fraley R. et al. in J. Biol. Chem. 255, 10431 (1980)). Most frequently used methods are the co-precipitation between DNA plus $Ca.PO_4$ (Graham, F.L. and van der Eb., A.J. in Virology 52, 465 (1973)) and DEAE-dextran mediated transfection (Vaheri A. et al. Virology 27, 435 (1965)).

After incubation the transfected cos-7 cells, the culture supernatants were collected to test for soluble $Fc_\epsilon R$ as follows:

The Cos cell supernatants were tested on an eosinophilic cell line, EoL-3 which expresses $Fc_\epsilon R$, the presence of such receptors have been tested utilising monoclonal antibodies specific to $Fc_\epsilon R$ (8-30) as described in European Patent Application No. 87110658.9 of the same applicant. As shown on Figure 5, these cells stain for $Fc_\epsilon R$ with 8-30 antibodies compared to the controls.

Furthermore, $Fc_\epsilon R$ on cells are detected by an assay with the use of fixed ox RBC (ORBS) coated with human IgE (Gonzalez-Molina, A. and Spiegelberg, H.L. J. Clin. Invest. 59, 616 (1977)). The number of IgE rosette-forming cells is estimated after subtracting the number of non-specific binding with fixed ORBC coated with bovine serum albumin.

The IgE-binding activity found in the PDE-2sFc$_\epsilon$R transfected Cos cell supernatant was analysed by its inhibitory activity on the binding (rosette-formation) of IgE-ORBC to the EoL-3 cells. The results shown on Table I indicate that the soluble Fc$_\epsilon$R is capable of inhibiting competitively the binding of IgE to its receptors.

The presence of soluble receptor activity was confirmed by the ELISA assay as described below:

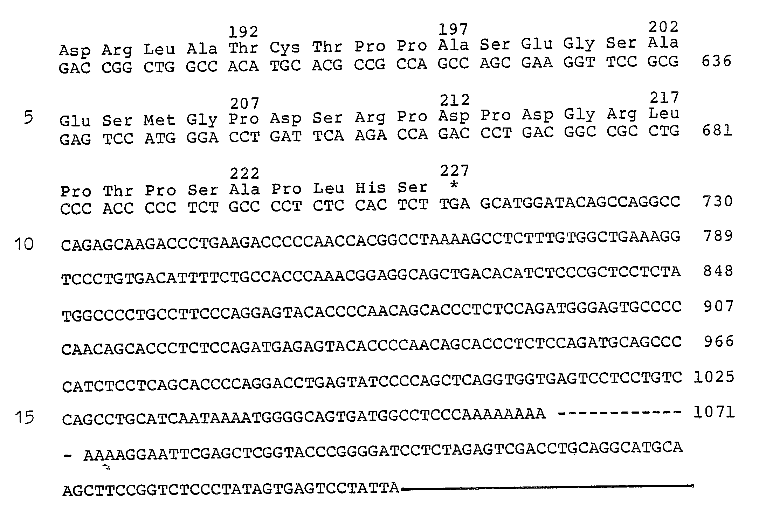

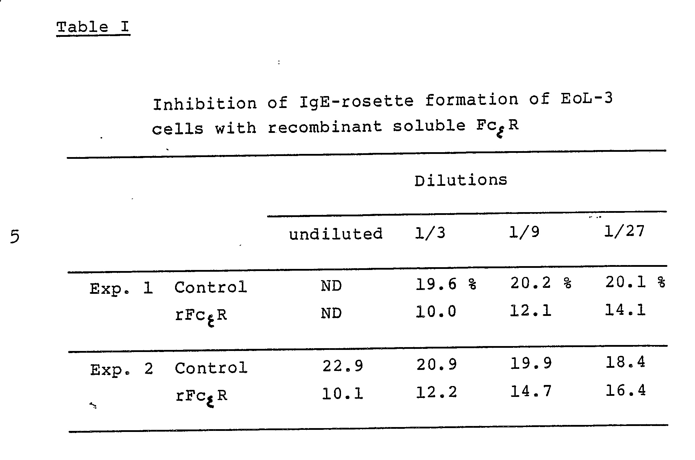

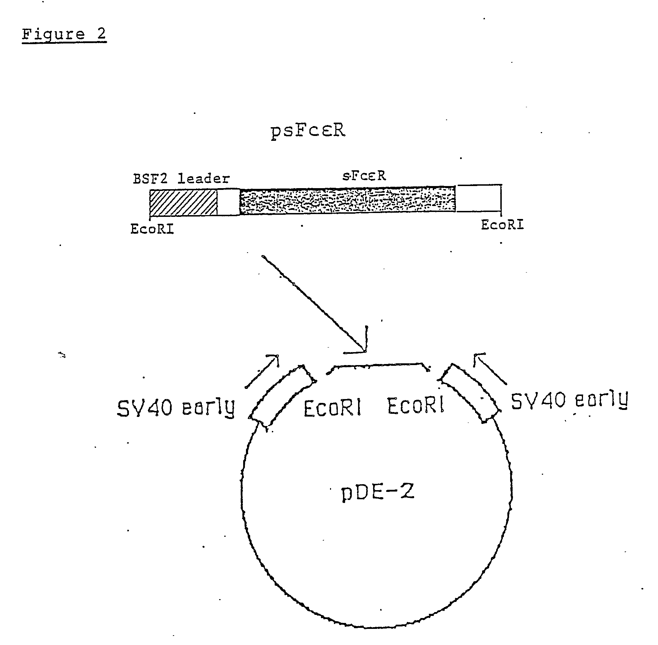

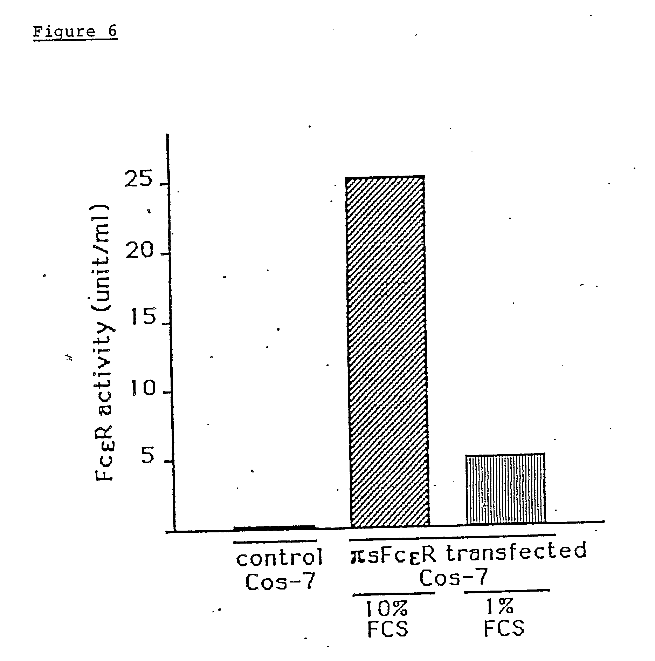

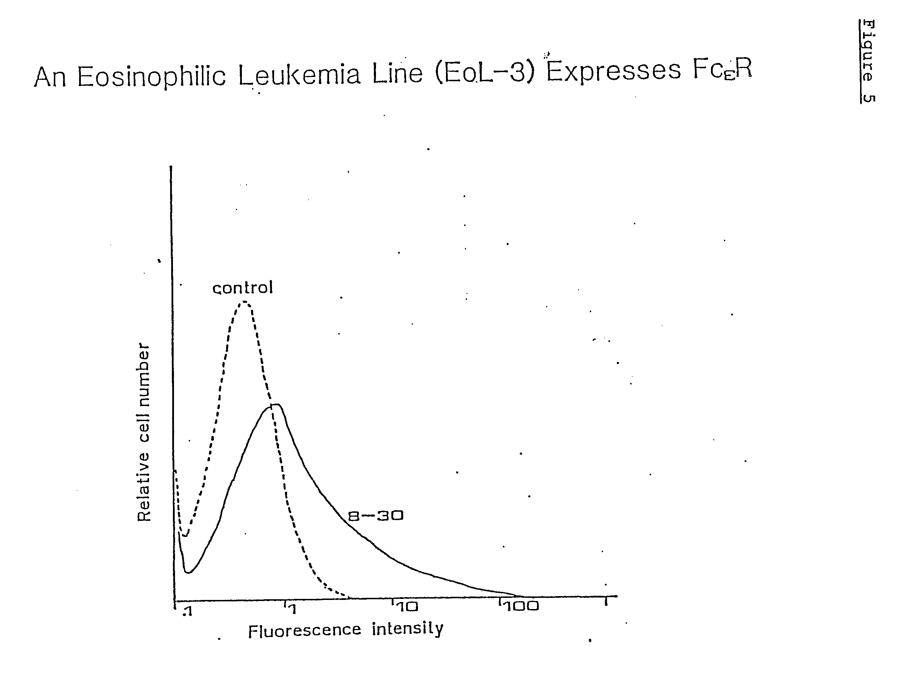

The Fc$_\epsilon$R activity was measured by its ability to bind to the monoclonal antibodies 3-5 and 8-30 and monitored utilising a double antibody enzyme-linked immunosorbent assay (ELISA). Whereas no Fc$_\epsilon$R activity was detected in the supernatants of control Cos-7 cells, there was significant secretion of receptor activity in the PDE-2sFc$_\epsilon$R-transfected Cos-7 cells. In fact, the level of Fc$_\epsilon$R activity was 5 and 25 units/ml in transfectant culture supernatants containing 1 % and 10 % fetal calf serum (FCS) respectively. The results of Fc$_\epsilon$R activity are shown on Figure 6.

Moreover, it is known, that immediate-type hypersensitivity can be manifested as a broad spectrum of symptoms ranging from seasonal rhinitis to anaphylaxis. The mechanism of immediate hypersensitivity involves IgE antibodies and reactive cells such as tissue mast cells and basophils which bear on their surface specific receptors for these antibodies. Upon triggering of those cells by specific allergens, a cascade of biochemical events lead to the release of preformed mediators from the cellular granules and newly synthesized lipid metabolites of membrane arachidonic acid, these agents cause an inflammatory process characterised by increased vascular permeability, smooth muscle contraction and all the symptoms associated with such a reaction. The process is amplified by the chemotaxis of cells such as neutrophils and eosinophils which in turn will enhance the reaction by re leasing their own proinflammatory components. A family of factors specific for the eosinophils are the eosinophil chemotactic factors of anaphylaxis, these are acidic tetrapeptides and attract eosinophils to the site of the allergic reaction. More recent findings indicate the importance of eosinophils in allergy and in parasitic infestations. It has been demonstrated that the eosinophils bear on their surface receptors for IgE (Fc$_\epsilon$R) which in the presence of IgE can actively participate in IgE-mediated events characterised by degranulation and further amplification of the inflammatory process. Furthermore, the Fc$_\epsilon$R found on eosinophils is of low affinity compared to that found on mast cells and basophils. In view of the mounting importance of eosinophils in immediate-type hypersensitivity and particularly asthma it is important to find means to control the IgE-mediated degranulation of these cells. Moreover, the central issue is to prevent or compete with the binding of IgE to mast cells, basophils and eosinophils. It has been hypothesised that IgE-binding factors might be able to achieve that either by controlling IgE biosynthesis or by neutralising or scavenging serum IgE.

The before mentioned evidence shows that the water-soluble Fc$_\epsilon$R prepared according to the present invention binds IgE and in its soluble form it can prevent the binding of IgE to eosinophils which are known to be involved in certain aspects of immediate hypersensitivity (allergy) and particularly asthma.

Therefore, the soluble Fc$_\epsilon$R prepared according to the invention is useful in controlling the inflammatory effects of allergy and is suitable for the treatment or prophylaxis of local and allergic reactions induced by IgE, which is a further object of the invention.

The soluble Fc$_\epsilon$R may be incorporated for the pharmaceutical use in the suitable pharmaceutical compositions, such as solutions or sprays.

The following examples, which are not exhaustive, will illustrate the invention in greater detail:

Example A

The monoclonal anti-Fc$_\epsilon$R antibodies 3-5 ($\gamma$·) and 8-30 ($\mu$) were produced by hybridization of P3UI myeloma with spleen cells from Balb/c mice immunized with RPMI-8866 cells (see European Patent Application No. 86 110 420.6 of the same Applicant, filed on July 29, 1986). The 8-30 antibody recognizes the epitope close to IgE binding site of Fc$_\epsilon$R and can block binding of IgE to 8866 lymphoblastoid cells. The 3-5 antibody, recognizes a different epitope on Fc$_\epsilon$R and can not block effectively IgE binding to its receptors. These antibodies precipitate 46 kd and 25 kd polypeptides under reducing and non reducing conditions. The monoclonal antibodies were purified from ascitis by 50 % saturated ammonium sulfate precipitation followed by gel filtration using Sepharose 6B (Pharmacia Fine Chemical, Uppsala, Sweden) for IgM class or ion exchange chromatography using QAE-Sephadex (Pharmacia Fine Chemical) for IgGl. The polyclonal mouse IgG was isolated in the same fashion. The anti-mouse IgM-alkaline phosphatase conjugate was purchased from Tago (Burlingame, CA).

Example B

Construction of plasmid psFc$_\epsilon$R-1

a) 350 μg of pBSF$_2$-38 (see Nature 324, 73-76 (1986)), which contains the BSF-2 cDNA in the SmaI site of pGEM4, were digested with 700 units of EcoRI and BamHI in 500 μl of a high salt buffer (100 mM NaCl, 50 mM Tris-HCl, pH 7.5, 10 mM MgC1$_2$, 1 mM DTT) for 2 hrs at 37°C. The digested DNA was applied on a preparative 1 % agarose gel electrophoresis and the EcoRI-BamHI fragment containing the full-length 1.2 kbp BSF-2 cDNA was electroeluted from the gel, precipitated with 70 % ethanol and dissolved at a concentration of 1 μg/μl in TE buffer. 20 μg of this fragment were digested with 40 units of HinfI in 50 μl of a high salt buffer for 1 hr, phenol-extracted and ethanol-precipitated. The digested DNAs were dissolved in 25 μl of 1 x nick translation buffer (50 mM Tris-HCl, pH 7.2, 10 mM MgSO$_4$, 0.1 mM DTT, 50 μg/ml BSA) and incubated with 1 ml of 8.0 units/μl Klenow fragment and 1 mM dNTP at 20°C for 30 minutes. The filling in reaction was terminated by incubation at 70°C for 5 min. The resulting 127 bp blunt ended fragment was phenol-extracted, digested with 40 units KpnI in 50 μl of a low salt buffer (10 mM Tris-HCl, pH 7.5, 10 mM MgCl$_2$, 1 mM DTT) for 1 hr at 37°C, incubated with 2.5 units bacterial alkaline phosphatase at 65°C for 30 min and then applied on 1 % preparative agarose gel and electrophoresed. The 110 bp fragment containing the BSF-2 leader sequence was electroeluted and ethanol-precipitated. - The 110 bp fragment was dissolved in 10 μl of ligation buffer (50 mM Tris-HCl, pH 7.4, 10 mM MgCl$_2$, 10 mM DTT, 1 mM spermidine, 1 mM ATP, 0,1 mg/ml BSA) and ligated with 1 μg of KpnI and SmaI digested PGEM4 by incubating with 200 units of T4 ligase at 4°C for 16 hrs and transfected into E.coli (MC1065). From the obtained colonies four colonies were picked up, one clone was selected, propagated and after confirmation that the plasmid of this selected clone contained only one leader sequence, it was named as pBSF2-L8 (see Figure 3).

b) 80 μg of plasmid LE-392 or PGEM4(pFc$_\epsilon$R-1) were digested with 150 units of HindIII in 200 μl of a low salt buffer (10 mM Tris-HCl, pH 7.5, 10 mM MgCl$_2$, 1 mM DTT) at 37°C for 1 hr and applied on 1 % preparative agarose gel electrophoresis. The HindIII fragment containing the soluble Fc R region was electroeluted from the gel, ethanol-precipitated and dissolved at a concentration of 1 μg/μl in TE buffer. 1 μg of the HindIII fragment was incubated with 8.2 units Klenow fragment and 1 mM dNTP in 10 μl of 1 x nick translation buffer at 20°C for 30 min to fill in the recessive 3'-ends, phenol-extracted and ethanol-precipitated. The HindIII fragments, the 3'-ends of which had been filled in, were digested with 2 units PstI in 10 μl of the medium salt buffer (50 mM NaCl, 10 mM Tris-HCl, pH 7.5, 10 mM MgCl$_2$, 1 mM DTT) at 37°C for 1 hr, incubated with 0.25 unit bacterial alkaline phosphatase at 65°C for 30 min and ethanol-precipitated. Separately, 1 μg of pBSF2-L8 was digested with 2 units BamHI in 20 μl of a high salt buffer at 37°C for 1 hr, phenol-extracted and ethanol-precipitated. The BamHIdigested pBSF2-L8 was dissolved in 10 μl of 1 x nick translation buffer and incubated with 8.0 units Klenow fragment and 1 mM dNTP at 20°C for 30 min to fill in the recessive 3'-ends, phenol-extracted and ethanol-precipitated. The precipitate was dissolved in 20 μl of a high salt buffer, digested with 2 units PstI at 37°C for 1 hr, phenol-extracted and ethanol-precipitated. - The PstI-digested fragment containing the soluble Fc$_\epsilon$R coding region and PstI digested pBSF2-L8 were ligated by incubating with 200 units T4 ligase in 10 μl of ligation buffer at 4°C for 16 hrs and transfected into E.coli (MC1065). Eight colonies were picked up from the obtained colonies. One clone was selected, propagated and after confirmation of the plasmid construction named psFc$_\epsilon$R-1. The propagated psFc R-1 contains seven bases from the multiple cloning into pGEM4 between BSF-2 leader and Fc$_\epsilon$R sequences in frame (see Figure 1: nucleotides 137 to 143).

Example 1

Construction of PDE-2sFc$_\epsilon$R

20 μg of the expression vector PDE-2 (see Japanese Patent Publication 1986/88879) were digested with 40 units of EcoRI in a total volume of 100 μl containing 50 mM NaCl, 100 mM Tris-HCl pH 7.5, 5 mM MgCl$_2$ and 100 μg/ml BSA at 37°C for two hours. Following extraction with phenol/chloroform the DNA was recovered by ethanol precipitation. The digested DNA was dissolved in 25 μl and was treated with 20 units of bacterial alkaline phosphatase at 65°C for 30 minutes and phenol/chloroform extracted twice and ethanol precipitated. In parallel an EcoRI fragment of psFc$_\epsilon$R-1 plasmid (see Figure 4) was produced as follows: 20 μg of psFc$_\epsilon$R-1 were digested with 20 units of EcoRI in 100 μl of 50 mM NaCl, 100 mM Tris-HCl pH 7.5, 5 mM MgCl$_2$ and 100 μg/ml BSA at 37°C for two hours. Both the vector and the insert DNA were applied on 1 % preparative agarosegel and electrophoresed. The 1,5 kb EcoRI

fragment of psFc$_\epsilon$R-1 (consisting of the BSF-2 leader and sFc$_\epsilon$-sequence) and the linearised PDE-2 vector were electroeluted and ethanol precipitated. The dephosphorylated linearised vector was ligated with the 1,5 kb EcoRI fragment by incubating with 200 units of T4 ligase in 20 $\mu$l of ligation buffer (50 mM Tris-HCl pH 7.4, 10 mM MgCl$_2$, 20 mM DTT, 1 mM spermidin, 1 mM ATP and 100 $\mu$g/ml BSA) at 4°C for 16 hours and transfected into E.coli. Restriction enzyme analysis was performed to identify the correct insert. One positive clone was selected and named as pDE-2sFc R. The resulting expression vector PDE-2sFc$_\epsilon$R was utilized for expression in monkey Cos-7 cells according to known methods.

Example 2

Expression of PDE-2sFc$_\epsilon$R

Cos-7 cells (5 x 10$^5$ cells per plate, diameter 60 mm) were seeded onto 60 mm plates one day prior to transfection. Transfection was carried out with 2 $\mu$l of plasmid DNA in 1 ml of 25 mM Tris-HCl (pH 7.5), 137 mM NaCl, 5 mM KCl, 0,6 mM Na$_2$HPO$_4$, 0,5 mM MgCl$_2$, 0,7 mM CaCl$_2$ and 500 $\mu$g of DEAE-dextran (Pharmacia Fine Chemical). After 1 hour incubation at 37°C, the solution was replaced with DMEM containing 10 % FCS and 150 $\mu$M chloroquine, incubated at 37°C for 3 hours and then replaced with fresh DMEM containing 10 % FCS. One day after transfection the medium was changed to contain 1 % or 10 % FCS in DMEM. The culture supernatants were collected 2 days following the medium change and tested for soluble Fc$_\epsilon$R activity.

Example 3

IgE rosette formation and its inhibition

For IgE rosette inhibition, 25 $\mu$l of Fc$_\epsilon$R bearing cells (5x10$^6$/ml) are mixed with a specific volume (e.g. 100 $\mu$l) of test sample or control medium and incubated for 1 hour at 4°C. The number of rosettes with 3 or more ORBC are counted. The results are shown in Table I. In experiments I and II the Fc$_\epsilon$R bearing cells are the EoL-3 cells. The control medium is the supernatant of the control Cos-7 cells, i.e. non-transformed cells.

Example 4

Enzyme Linked Immunosorbent Assay (ELISA)

96 well mictrotiter plates were initially coated with the monoclonal antibody 3-5 100 $\mu$l well (10 $\mu$g/ml) in coating buffer (NaHCO$_3$ 0.1 M, pH 9.6), and incubated overnight at 4°C.
The plates were then washed 4 times with rinse buffer, i.e. Dulbecco's phosphate buffer pH containing 0,05 % Tween 20, followed by the addition of 100 $\mu$l test sample diluted with diluent buffer (Tris-HCl 0,05 M, pH 8.1, MgCl$_2$ 1 mM, NaCl 0,15 M, Tween 20 0,05 % (v/v), NaN$_3$ 0,02 %, BSA 1 %). The microtiter plates were incubated for 2 hours at room temperature, and washed 4 times with rinse buffer, followed by the addition of 100 $\mu$l of pretitrated and diluted goat-anti-mouse IgM-alkaline phosphatase conjugates. The plates were incubated for two hours at room temperature and washed 4 times with rinse buffer. In the final step 100 $\mu$l of substrate, p-phenyl phosphate disodium (1 mg/ml) in substrate buffer (NaHCO$_3$ 0,05M, pH 9.8, MgCl$_2$ x 6 H$_2$O, 10 mM) was added and the colorimteric reaction measured every 30 minutes for two hours at 405 and 620 nm. The results of Fc$_\epsilon$R activity are shown on Figure 6.

**Claims**

1. A cloned gene operably linked to a replicon and control sequence both suitable for expression in cells of multicellular organisms wherein at least a part of the coding sequence for the amino acids 1 to 148 of the whole Fc$_\epsilon$R-gene as shown in Figure 1 is replaced by an eucaryotic signal sequence or a coding equivalent thereof.

2. The cloned gene of claim 1 wherein said signal sequence is an interleukin cDNA signal sequence or a coding equivalent thereof.

3. The cloned gene of claim 2 wherein said signal sequence is the BSF-2 signal sequence or a coding equivalent thereof.

4. The cloned gene of claim 3 wherein said BSF-2 signal sequence is comprised by the 110 bp KpnI-HinfI fragment of the plasmid pBSF-2.38 or a coding equivalent thereof.

5. The cloned gene of claim 1 showing the formula as shown in Figure 4 or a coding equivalent thereof.

6. An expression vector suitable for expression in cells of multicellular organisms containing a cloned gene as claim in any of the claims 1 to 5 or a coding equivalent thereof.

7. An expression vector as claimed in claim 6 wherein as basic vector is used the PDE-2sFc$_\epsilon$R as shown in Figure 2.

8. A cell of a multicellular organism transfected with an expression vector as claimed in claim 6 or 7.

9. A cell of a multicellular organism of claim 8 wherein said cell is one of a vertibrate cell line.

10. A cell of a multicellular organism of claim 9 wherein said vertibrate cell is one of Cos-7 cells.

11. Water-soluble part of human low affinity $Fc_\epsilon$-receptor coded by a DNA-molecule as claimed in any of the claims 1 to 5.

12. Water-soluble part of human low affinity $Fc_\epsilon$-receptor as claimed in claim 11 wherein said $Fc_\epsilon$-receptor is expressed by a cell line as claimed in any of the claims 8 to 10.

13. Water-soluble part of human low affinity $Fc_\epsilon$-receptor as claimed claim 11 or 12 containing at least the amino acids 150 to 321 as shown in Figure 1.

14. Water-soluble part of human low affinity $Fc_\epsilon$-receptor as claimed in any of the claims 11 to 13 containing the BSF-2.38 leader peptide and the amino acids 134 to 321 as shown in Figure 1.

15. Water-soluble part of human low affinity $Fc_\epsilon$-receptor as claimed in claim 14 wherein the expressed peptide has the formula as shown in Figure 4.

16. The 0-glycosylated derivate of the water-soluble part of human low-affinity $Fc_\epsilon$-receptor as claimed in any of the claims 11 to 15.

17. Pharmaceutical composition containing a water-soluble $Fc_\epsilon$-receptor as claimed in any of the claims 11 to 16.

18. Pharmaceutical compositions as claimed in claim 17 suitable for treatment of local or systemic IgE-allergic reactions.

19. Use of the water-soluble $Fc_\epsilon$-receptor as claimed in any of the claims 11 to 16 for the preparation of a pharmaceutical composition.

20. A method of preparing a cloned gene as claimed in any of the claims 1 to 5 which comprises ligating a DNA-sequence coding at least the water-soluble part of human low affinity receptor starting with the amino acid 150 as shown in Figure 1 with an eucaryotic signal sequence.

21. A method of preparing an expression vector as claimed in claim 6 or 7 which comprises ligating a suitable linearised vector with a cloned gene as claim in any of the claims 1 to 5.

22. A method of preparing a cell line as claimed in any of the claims 8 to 10 which comprises transfecting a cell with an expression vector as claimed in claim 6 or 7.

23. A method of preparing water-soluble human low affinity $Fc_\epsilon$-receptor as claimed in any of the claims 11 to 16 comprising culturing a transfected cell as claimed in any of the claims 8 to 10 and isolating the water-soluble $Fc_\epsilon$-receptor as produced.

24. Preparation of a pharmaceutical composition as claimed in claim 17 or 18 which comprises incorporating an effective amount of a water-soluble $Fc_\epsilon$-receptor as claimed in any of the claims 11 to 16 in one or more excipients.

## Figure 1: Scheme of pFcₑR-1

```
                                                        EcoRI
                                                      ── GAATTCCCTCCTGCT        8

TAAACCTCTGTCTCTGACGGTCCCTGCCAATCGCTCTGGTCGACCCCAACACACTAGGA        67

GGACAGACACAGGCTCCAAACTCCACTAAGTGACCAGAGCTGTGATTGTGCCCGCTGAG       126

TGGACTGCGTTGTCAGGGAGTGAGTGCTCCATCATCGGGAGAATCCAAGCAGGACCGCC       185

                        5                    10                  15
    Met Glu Glu Gly Gln Tyr Ser Glu Ile Glu Glu Leu Pro Arg Arg
    ATG GAG GAA GGT CAA TAT TCA GAG ATC GAG GAG CTT CCC AGG AGG       230

                            20                  25              30
    Arg Cys Cys Arg Arg Gly Thr Gln Ile Val Leu Leu Gly Leu Val
    CGG TGT TGC AGG CGT GGG ACT CAG ATC GTG CTG CTG GGG CTG GTG       275

                        35                  40                  45
    Thr Ala Ala Leu Trp Ala Gly Leu Leu Thr Leu Leu Leu Leu Trp
    ACC GCC GCT CTG TGG GCT GGG CTG CTG ACT CTG CTT CTC CTG TGG       320

                        50                  55              60
    His Trp Asp Thr Thr Gln Ser Leu Lys Gln Leu Glu Glu Arg Ala
    CAC TGG GAC ACC ACA CAG AGT CTA AAA CAG CTG GAA GAG AGG GCT       365

                        65                  70              75
    Ala Arg Asn Val Ser Gln Val Ser Lys Asn Leu Glu Ser His His
    GCC CGG AAC GTC TCT CAA GTT TCC AAG AAC TTG GAA AGC CAC CAC       410

                        80                  85              90
    Gly Asp Gln Met Ala Gln Lys Ser Gln Ser Thr Gln Ile Ser Gln
    GGT GAC CAG ATG GCG CAG AAA TCC CAG TCC ACG CAG ATT TCA CAG       455

                        95                  100             105
    Glu Leu Glu Glu Leu Arg Ala Glu Gln Gln Arg Leu Lys Ser Gln
    GAA CTG GAG GAA CTT CGA GCT GAA CAG CAG AGA TTG AAA TCT CAG       500

                        110                 115             120
    Asp Leu Glu Leu Ser Trp Asn Leu Asn Gly Leu Gln Ala Asp Leu
    GAC TTG GAG CTG TCC TGG AAC CTG AAC GGG CTT CAA GCA GAT CTG       545

                        125                 130             135
    Ser Ser Phe Lys Ser Gln Glu Leu Asn Glu Arg Asn Glu Ala Ser
    AGC AGC TTC AAG TCC CAG GAA TTG AAC GAG AGG AAC GAA GCT TCA       590

                        140                 145             150
    Asp Leu Leu Glu Arg Leu Arg Glu Glu Val Thr Lys Leu Arg Met
    GAT TTG CTG GAA AGA CTC CGG GAG GAG GTG ACA AAG CTA AGG ATG       635

                        155                 160             165
    Glu Leu Gln Val Ser Ser Gly Phe Val Cys Asn Thr Cys Pro Glu
    GAG TTG CAG GTG TCC AGC GGC TTT GTG TGC AAC ACG TGC CCT GAA       680
```

```
            170                    175              180
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC   725

            185                    190              195
Thr Lys Gln Trp Val His Ala Arg Tyr Ala Cys Asp Asp Met Glu
ACC AAG CAG TGG GTC CAC GCC CGG TAT GCC TGT GAC GAC ATG GAA   770

            200                    205              210
Gly Gln Leu Val Ser Ile His Ser Pro Glu Glu Gln Asp Phe Leu
GGG CAG CTG GTC AGC ATC CAC AGC CCG GAG GAG CAG GAC TTC CTG   815

            215                    220              225
Thr Lys His Ala Ser His Thr Gly Ser Trp Ile Gly Leu Arg Asn
ACC AAG CAT GCC AGC CAC ACC GGC TCC TGG ATT GGC CTT CGG AAC   860

            230                    235              240
Leu Asp Leu Lys Gly Glu Phe Ile Trp Val Asp Gly Ser His Val
TTG GAC CTG AAG GGA GAG TTT ATC TGG GTG GAT GGG AGC CAT GTG   905

            245                    250              255
Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG   950

            260                    265              270
Gly Glu Asp Cys Val Met Met Arg Gly Ser Gly Arg Trp Asn Asp
GGC GAG GAC TGC GTG ATG ATG CGG GGC TCC GGT CGC TGG AAC GAC   995

            275                    280              285
Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG  1040

            290                    295              300
Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG  1085

            305                    310              315
Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC  1130

            320
Ser Ala Pro Leu His Ser    *
TCT GCC CCT CTC CAC TCT TGA GCATGGATACAGCCAGGCCCAGAGCAAGACC  1182

CTGAAGACCCCCAACCACGGCCTAAAAGCCTCTTTGTGGCTGAAAGGTCCCTGTGACAT  1241

TTTCTGCCACCCAAACGGAGGCAGCTGACACATCTCCCGCTCCTCTATGGCCCCTGCCT  1300

TCCCAGGAGTACACCCCAACAGCACCCTCTCCAGATGGGAGTGCCCCCAACAGCACCCT  1359

CTCCAGATGAGAGTACACCCCAACAGCACCCTCTCCAGATGCAGCCCCATCTCCTCAGC  1418
```

ACCCCAGGACCTGAGTATCCCCAGCTCAGGTGGTGAGTCCTCCTGTCCAGCCTGCATCA 1477

ATAAAATGGGGCAGTGATGGCCTCCCAAAAA ------------------------- 1507

----- AAGGAATTC /Sac/Kpn/    /Pst/Sph/Hind/ ━━━━━━━
             EcoRI

## Figure 3: Scheme of pBSF2-L8

```
                                                          -25
                                  KpnI   Met Asn Ser Phe
_____/Eco/Sac/GGTACC ATG AAC TCC TTC   18

         -20                          -15              -10
Ser Thr Ser Ala Phe Gly Pro Val Ala Phe Ser Leu Gly Leu Leu
TCC ACA AGC GCC TTC GGT CCA GTT GCC TTC TCC CTG GGG CTG CTC   63

              -5                    1
Leu Val Leu Pro Ala Ala Phe Pro Ala Pro Val Pro Pro Gly Glu      6
CTG GTG TTG CCT GCT GCC TTC CCT GCC CCA GTA CCC CCA GGA GAA   108

              11                   16                   21
Asp Ser Lys Asp Val Ala Ala Pro His Arg Gln Pro Leu Thr Ser
GAT TCC AAA GAT GTA GCC GCC CCA CAC AGA CAG CCA CTC ACC TCT   153

              26                   31                   36
Ser Glu Arg Ile Asp Lys Gln Ile Arg Tyr Ile Leu Asp Gly Ile
TCA GAA CGA ATT GAC AAA CAA ATT CGG TAC ATC CTC GAC GGC ATC   198

              41                   46                   51
Ser Ala Leu Arg Lys Glu Thr Cys Asn Lys Ser Asn Met Cys Glu
TCA GCC CTG AGA AAG GAG ACA TGT AAC AAG AGT AAC ATG TGT GAA   243

              56                   61                   66
Ser Ser Lys Glu Ala Leu Ala Glu Asn Asn Leu Asn Leu Pro Lys
AGC AGC AAA GAG GCA CTG GCA GAA AAC AAC CTG AAC CTT CCA AAG   288

              71                   76                   81
Met Ala Glu Lys Asp Gly Cys Phe Gln Ser Gly Phe Asn Glu Glu
ATG GCT GAA AAA GAT GGA TGC TTC CAA TCT GGA TTC AAT GAG GAG   333

              86                   91                   96
Thr Cys Leu Val Lys Ile Ile Thr Gly Leu Leu Glu Phe Glu Val
ACT TGC CTG GTG AAA ATC ATC ACT GGT CTT TTG GAG TTT GAG GTA   378

              101                  106                  111
Tyr Leu Glu Tyr Leu Gln Asn Arg Phe Glu Ser Ser Glu Glu Gln
TAC CTA GAG TAC CTC CAG AAC AGA TTT GAG AGT AGT GAG GAA CAA   423

              116                  121                  126
Ala Arg Ala Val Gln Met Ser Thr Lys Val Leu Ile Gln Phe Leu
GCC AGA GCT GTG CAG ATG AGT ACA AAA GTC CTG ATC CAG TTC CTG   468

              131                  136                  141
Gln Lys Lys Ala Lys Asn Leu Asp Ala Ile Thr Thr Pro Asp Pro
CAG AAA AAG GCA AAG AAT CTA GAT GCA ATA ACC ACC CCT GAC CCA   513

              146                  151                  156
Thr Thr Asn Ala Ser Leu Leu Thr Lys Leu Gln Ala Gln Asn Gln
ACC ACA AAT GCC AGC CTG CTG ACG AAG CTG CAG GCA CAG AAC CAG   558
```

EP 0 321 601 A1

```
                      161                 166                 171
Trp Leu Gln Asp Met Thr Thr His Leu Ile Leu Arg Ser Phe Lys
TGG CTG CAG GAC ATG ACA ACT CAT CTC ATT CTG CGC AGC TTT AAG   603

                      176                 181
Glu Phe Leu Gln Ser Ser Leu Arg Ala Leu Arg Gln Met
GAG TTC CTG CAG TTC AGC CTG AGG GCT CTT CGG CAA ATG TAGCATG   649

GGCACCTCAGATTGTTGTTGTTAATGGGCATTCCTTCTTCTGGTCAGAAACCTGTCCAC   708

TGGGCACAGAACTTATGTTGTTCTCTATGGAGAACTAAAAGTATGAGCGTTAGGACACT   767

ATTTTAATTATTTTTAATTTATTAATATTTAAATATGTGAAGCTGAGTTAATTTATGTA   826

AGTCATATTTATATTTTAAGAAGTACCACTTGAAACATTTTATGTATTAGTTTTGAAAT   885

AATAATGGAAAGTGGCTATGCAGTTTGAATATCCTTTGTTTCAGAGCCAGATCATTTCT   944

TGGAAAGTGTAGGCTTACCTCAAATAAATGGCTAACTTATACATATTTTTAAAGAAATA  1003

TTTATATTGTATTTATATAATGTATAAATGGTTTTTATACCAATAAATGGCATTTTAAA  1062

AAATTCAGCAAAAAAAAAAAAAAAAAAAAAAAAAGGGATCC/Xba/SaI/Pst/Sph/Hin/1100
                                          BamHI
```

Figure 4: Scheme of psFcεR-1

——————————————————————————————————— ATTTAGGTGACACTATA

```
                                         2                       7
              EcoRI        Kpnl        Met Asn Ser Phe Ser Thr Ser
    GAATACACGGAATTCGAGCTCGGTACCCCT ATG AAC TCC TTC TCC ACA ACC      51

                         12                  17                  22
    Ala Phe Gly Pro Val Ala Phe Ser Leu Gly Leu Leu Leu Val Leu
    GCC TTC GGT CCA GTT GCC TTC TCC CTG GGG CTG CTC CTG GTG TTG      96

                         27                  32                  37
    Pro Ala Ala Phe Pro Ala Pro Val Pro Pro Gly Glu Asp Trp Gly
    CCT GCT GCC TTC CCT GCC CCA GTA CCC CCA GGA GAA GAT TGG GGA     141

                         42                  47                  52
    Ser Ala Ser Asp Leu Leu Glu Arg Leu Arg Glu Glu Val Thr Lys
    TCA GCT TCA GAT TTG CTG GAA AGA CTC CGG GAG GAG GTG ACA AAG     186

                         57                  62                  67
    Leu Arg Met Glu Leu Gln Val Ser Ser Gly Phe Val Cys Asn Thr
    CTA AGG ATG GAG TTG CAG GTG TCC AGC GGC TTT GTG TGC AAC ACG     231

                         72                  77                  82
    Cys Pro Glu Lys Trp Ile Asn Phe Gln Arg Lys Cys Tyr Tyr Phe
    TGC CCT GAA AAG TGG ATC AAT TTC CAA CGG AAG TGC TAC TAC TTC     276

                         87                  92                  97
    Gly Lys Gly Thr Lys Gln Trp Val His Ala Arg Tyr Ala Cys Asp
    GGC AAG GGC ACC AAG CAG TGG GTC CAC GCC CGG TAT GCC TGT GAC     321

                         102                 107                 112
    Asp Met Glu Gly Gln Leu Val Ser Ile His Ser Pro Glu Glu Gln
    GAC ATG GAA GGG CAG CTG GTC AGC ATC CAC AGC CCG GAG GAG CAG     366

                         117                 122                 127
    Asp Phe Leu Thr Lys His Ala Ser His Thr Gly Ser Trp Ile Gly
    GAC TTC CTG ACC AAG CAT GCC AGC CAC ACC GGC TCC TGG ATT GGC     411

                         132                 137                 142
    Leu Arg Asn Leu Asp Leu Lys Gly Glu Phe Ile Trp Val Asp Gly
    CTT CGG AAC TTG GAC CTG AAG GGA GAG TTT ATC TGG GTG GAT GGG     456

                         147                 152                 157
    Ser His Val Asp Tyr Ser Asn Trp Ala Pro Gly Glu Pro Thr Ser
    AGC CAT GTG GAC TAC AGC AAC TGG GCT CCA GGG GAG CCC ACC AGC     501

                         162                 167                 172
    Arg Ser Gln Gly Glu Asp Cys Val Met Met Arg Gly Ser Gly Arg
    CGG AGC CAG GGC GAG GAC TGC GTG ATG ATG CGG GGC TCC GGT CGC     546

                         177                 182                 187
    Trp Asn Asp Ala Phe Cys Asp Arg Lys Leu Gly Ala Trp Val Cys
    TGG AAC GAC GCC TTC TGC GAC CGT AAG CTG GGC GCC TGG GTG TGC     591
```

```
                            192                          197                        202
        Asp Arg Leu Ala Thr Cys Thr Pro Pro Ala Ser Glu Gly Ser Ala
        GAC CGG CTG GCC ACA TGC ACG CCG CCA GCC AGC GAA GGT TCC GCG    636

                            207                          212                        217
        Glu Ser Met Gly Pro Asp Ser Arg Pro Asp Pro Asp Gly Arg Leu
        GAG TCC ATG GGA CCT GAT TCA AGA CCA GAC CCT GAC GGC CGC CTG    681

                            222                          227
        Pro Thr Pro Ser Ala Pro Leu His Ser  *
        CCC ACC CCC TCT GCC CCT CTC CAC TCT TGA GCATGGATACAGCCAGGCC    730

        CAGAGCAAGACCCTGAAGACCCCCAACCACGGCCTAAAAGCCTCTTTGTGGCTGAAAGG    789

        TCCCTGTGACATTTTCTGCCACCCAAACGGAGGCAGCTGACACATCTCCCGCTCCTCTA    848

        TGGCCCCTGCCTTCCCAGGAGTACACCCCAACAGCACCCTCTCCAGATGGGAGTGCCCC    907

        CAACAGCACCCTCTCCAGATGAGAGTACACCCCAACAGCACCCTCTCCAGATGCAGCCC    966

        CATCTCCTCAGCACCCCAGGACCTGAGTATCCCCAGCTCAGGTGGTGAGTCCTCCTGTC   1025

        CAGCCTGCATCAATAAAATGGGGCAGTGATGGCCTCCCAAAAAAAA -----------   1071

        - AAAAGGAATTCGAGCTCGGTACCCGGGGATCCTCTAGAGTCGACCTGCAGGCATGCA

        AGCTTCCGGTCTCCCTATAGTGAGTCCTATTA
```

Table I

Inhibition of IgE-rosette formation of EoL-3 cells with recombinant soluble $Fc_\varepsilon R$

| | | Dilutions | | | |
|---|---|---|---|---|---|
| | | undiluted | 1/3 | 1/9 | 1/27 |
| Exp. 1 | Control | ND | 19.6 % | 20.2 % | 20.1 % |
| | $rFc_\varepsilon R$ | ND | 10.0 | 12.1 | 14.1 |
| Exp. 2 | Control | 22.9 | 20.9 | 19.9 | 18.4 |
| | $rFc_\varepsilon R$ | 10.1 | 12.2 | 14.7 | 16.4 |

Figure 2

## Figure 6

An Eosinophilic Leukemia Line (EoL-3) Expresses FcₑR

Figure 5

EP 0 321 601 A1

European Patent Office

**EUROPEAN SEARCH REPORT**

Application Number

EP 87 11 9080

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E,D | EP-A-0 259 615 (PROF. T. KISHIMOTO) <br> * Examples 13,14; figures 19,20 * <br><br> --- | 1-5,11-20,23, 24 | C 12 N  15/00 <br> C 12 N   5/00 <br> A 61 K  37/02 <br> C 12 P  21/02 |
| Y,D | CELL, vol. 47, 5th December 1986, pages 657-665, Cell Press; H. KIKUTANI et al.: "Molecular structure of human lymphocyte receptor for immunoglobulin E" <br> * Whole article; especially figure 4 * <br> --- | 1-24 | |
| Y,D | NATURE, vol. 324, no. 6092, 6th-12th November 1986, pages 73-76, Neptune, NJ, US; T. HIRANO et al.: "Complementary DNA for a novel human interleukin (BSF-2) that induces B lymphocytes to produce immunoglobulin" <br> * Whole article; especially page 75 * <br> ----- | 1-24 | |
| | | | **TECHNICAL FIELDS SEARCHED (Int. Cl.4)** <br><br> C 12 N <br> C 12 P |

The present search report has been drawn up for all claims

| Place of search | Date of completion of the search | Examiner |
|---|---|---|
| THE HAGUE | 19-08-1988 | CUPIDO M. |